# EUROPÄISCHE PATENTANMELDUNG

(11) **EP 1 346 733 A1**
(43) Veröffentlichungstag der Anmeldung: **24.09.2003**
(21) Anmeldenummer: 03003801.2
(22) Anmeldetag: 20.02.2003
(51) Int. Cl.: A61L 9/012, A61L 9/014, A61L 9/04, B01D 53/02, A61L 9/16

(54) **Verfahren zum Vernichten von schädlichen Mikroorganismen**

(30) Priorität: 14.03.2002 DE 10211165
(71) Anmelder: Air & D-Sarl, 67190 Rosheim (FR)
(72) Erfinder: Wuest, Robert, 67190 Rosheim (FR)
(74) Vertreter: Grussdorf, Jürgen, Dr.

(57) **Zusammenfassung**

Die Erfindung betrifft ein Verfahren zum Vernichten von schädlichen Mikroorganismen durch Behandeln der Luft mit bioziden aktiven Agentien, die in einer Matrix aus einem vernetzten, hydrophile Gruppen enthaltenden Polymeren verteilt sind.

## Beschreibung

Die Erfindung betrifft ein Verfahren zum Entfernen von schädlichen Mikroorganismen in der Luft durch Behandeln der Luft mit aktiven Agentien, die mit den Mikroorganismen reagieren und diese vernichten.

In schwierig zu belüftenden geschlossenen Räumen, wie Turnhallen, Hotelzimmern, Restaurants, Konferenzräumen und insbesondere in Krankenhäusern und Altersheimen sammeln sich schädliche Mikroorganismen an, die zu Infektionskrankheiten und Allergien führen können. In Befeuchtungsanlagen der genannten Räume ist dieses Problem besonders gravierend. An den Wänden von Luftschächten und Klimaanlagen lagern sich oft Staubschichten ab, in denen sich die Mikroorganismen festsetzen können. Es besteht daher ein Bedürfnis, ein einfaches aber wirksames Verfahren zur Vernichtung dieser Mikroorganismen bereitzustellen.

Diese Aufgabe wird gelöst durch Behandeln der Luft mit aktiven Agentien, die mit den Mikroorganismen reagieren und diese vernichten, wobei die aktiven Agentien erfindungsgemäß in einer Matrix aus einem vernetzten, hydrophile Gruppen enthaltenden Polymeren verteilt sind.

Beispiele für schädliche Mikroorganismen sind Viren, Pilze, Milben und insbesondere Bakterien, wie z.B. staphylococcus aureus, pseudomonas aeruginosa, escheria coli, mycobacterium smematis, bacillus subtilus, candida albicans, penicillium verrucosum und insbesondere legionella.

Geeignete Matrix- Polymere sind vernetzte maleinisierte oder epoxidierte Polymere und vernetzte (Meth-)Acrylat- Polymere.

Bevorzugt sind Kondensationsprodukte aus einem maleinisierten oder epoxidierten Polymeren und einem Vernetzer, vorzugsweise einem Polyamin. Geeignete Polymere sind Umsetzungsprodukte aus einem Polydien, z.B. Polybutadien, Polyoctadien und Soyabohnenöl, mit Maleinsäureanhydrid, ferner Copolymere von Olefinen, wie Ethylen, mit Maleinsäureanhydrid, sowie epoxidiertes Polybutadien. Bevorzugte Vernetzer sind Polyamine, insbesondere Polyoxypropylendiamin und Polyoxypropylentriamin. Daneben sind auch Harnstoff, Polyethylenimin und Triethylenglykol als Vernetzer geeignet. Die Vernetzungsreaktion erfolgt vorzugsweise in alkoholischer Lösung, z.B. in Dipropylenglykol, bei erhöhter Temperatur. Als hydrophile Gruppen wirken insbesondere die von den Polyoxyalkylenpolyaminen stammenden -CRH-O- Gruppen, daneben auch die Maleinsäureanhydrid- und Carboxylgruppen oder Epoxidgruppen bzw. die -NR-CO- Gruppen des vernetzten Polymeren.

Eine andere Klasse von vernetzten Polymeren sind Copolymerisate von monofunktionellen (Meth-)Acrylat- Monomeren, z.B. Hydroxyethylacrylat oder Poly(propylenoxid)(ethylenoxid)monomethacrylat, mit einem polyfunktionellen (Meth-)Acrylat- Monomeren, z. B. Ethylenglykoldimethacrylat oder Polyethylenglykol-400- dimethacrylat. Die Herstellung der vernetzten (Meth-)Acrylat- Polymeren erfolgt durch radikalische Copolymerisation der Monomeren.

In beiden Fällen sind die vernetzten Polymeren in der Lage, Flüssigkeiten und Gase, z.B. die flüchtigen Agentien zu absorbieren, wobei sich eine offenzellige Schwammstruktur ausbildet. Das vernetzte Polymere weist ein räumliches Netzwerk mit Poren auf, in dem flüchtige Fremdsubstanzen aufgesaugt und absorbiert werden können, so dass das Polymere wie ein Schwamm voluminös anquillt. Im angequollenen Zustand besteht das dreidimensionale Netzwerk aus Elementarzellen die im Mittel ein Volumen von 1 bis 1000 nm³, vorzugsweise von 3 bis 200 nm³, aufweisen.

Das vernetzte Polymere ist erfindungsgemäß mit einem bioziden aktiven Agens beladen und bildet mit diesem zusammen eine schwammartige Masse. Das aktive Agens wird daraus langsam freigesetzt.

Die aktiven Agentien sind flüchtige, vorzugsweise bakterizid wirkende Substanzen, z.B. Phenolverbindungen, wie Tetranyl der Fa. KAO Comp., Halogenverbindungen, wie Ammoniumjodid, quarternäre Ammoniumverbindungen, Aldehyde, natürliche und synthetische Essenzen und stabilisiertes Wasserstoffperoxid, mit denen das Polymere beladen ist und eine schwammartige Masse bildet. Aus dieser werden sie langsam freigesetzt, so dass sie in der Gasphase mit den Mikroorganismen reagieren und diese vernichten können. In Luftschächten und Klimaanlagen streicht die mit den aktiven Agentien beladene Luft über die Oberfläche der abgelagerten Staubschichten und reagiert dort mit den Mikroorganismen. Bevorzugt werden die flüchtigen aktiven Agentien bei der Herstellung der vernetzten Polymeren durch Kondensation bzw. Polymerisation zugesetzt, man kann aber auch das vernetzte Polymere mit ihnen tränken und damit anquellen. Die aktiven Agentien sollten in der schwammartigen Masse in Mengen von 10 bis 90 Gew.%, vorzugsweise von 40 bis 80 Gew.% enthalten sein.

Die schwammartige Masse kann neben der Polymermatrix und dem aktiven Agens noch weitere Zusatzstoffe, z.B. Wasser, Sublimationshilfsmittel und Flammschutzmittel enthalten.

Wesentlich ist, dass die aktiven Agentien mit den vernetzten Polymeren so abgestimmt sind, dass sie nur sehr langsam und während des Wirkungszeitraums gleichmäßig aus der schwammartigen Masse freigesetzt werden und ihre Wirkung mindestens drei Wochen, vorzugsweise mindestens zwei Monate und insbesondere mehr als drei Monate lang beibehalten. Im Gegensatz zu herkömmlichen Biozid- Sprays sind die erfindungsgemäß freigesetzten aktiven Agentien in sehr niedriger Konzentration wirksam und daher unschädlich. Ihre Wirkung ist jedoch konstant lange anhaltend.

Die schwammartige Masse, welche die aktiven Agentien enthält, kann in Form von Kugeln, Spänen oder Granulat zur Anwendung kommen. Sie wird jedoch bevorzugt in Form von Krümeln, Platten oder Streifen, vorzugsweise mit einer Dicke von 0,2 bis 5 cm, insbesondere von 0,5 bis 3 cm, eingesetzt. Die Kugeln, Späne oder Granulate können in Hülsen eingelegt werden, die an ihrem Umfang eine Vielzahl von Löchern aufweisen. Bevorzugt werden sie aber als Krümel, Platten oder Streifen auf Netze oder Gitter aufgelegt. Besonders günstig ist es, dabei mehrere Gitter übereinander auf einem Gestell anzuordnen und dieses in einen Ventilationskasten oder Klimakasten einzubauen, der an der Be- oder Entlüftung des Raums angebracht ist. An der Belüftung bewirkt die angesaugte Frischluft die Verdunstung der flüchtigen aktiven Agentien aus der schwammartigen Masse. Man kann die auf Gitter oder Netze aufgelegten Krümel, Streifen oder Platten auch in Lüftungsschächte einlegen, wo die verschmutzten Innenflächen, an denen sich Bakterien entwickeln könnten, entkeimen.

In Räumen mit Zwangsbelüftung können die Behältnisse mit der schwammartigen Masse direkt vor dem Lüftungsgitter angebracht werden, sonst über Eingangstüren, Kippfenstern oder Oberlichtern. Aufgrund der lang anhaltenden Wirkung der erfindungsgemäßen Einsätze muss die schwammartige Masse nur alle fünf bis zwölf Wochen ausgewechselt werden.

### Beispiel 1

21 g maleinisiertes Polybutadien (Umsetzungsprodukt von flüssigem Polybutadien mit Maleinsäureanhydrid - LITHENE der Fa. Revertex) wurden mit 79 g eines flüssigen handelsüblichen Biozids (TETRANYL der Fa. Kao Comp.) bei 45°C vermischt (Mischung A). 94 g des Biozids und 7,5 g Polyoxypropylentriamin (MG 400) wurden vermischt (Mischung B). Die Mischungen A und B wurden zusammengerührt.

Die erhaltene schwammartige Masse wurde in Platten von 1 cm Dicke, 15 cm Breite und 20 cm Länge geschnitten und auf ein Metallgitter aufgelegt. Mehrere solcher Gitter wurden übereinander auf einem Gestell angeordnet. Dieses Gestell wurde vor dem Lufteintrittsgitter eines Hotelzimmers angebracht, wo es über drei Monate hinweg ihre biozide Wirkung entfaltete.

### Beispiel 2

In einem geschlossenen Gefäß wurden 4 g Poly(propylenoxid)(ethylenoxid)-Monomethylacrylat mit 0,5 g Polyethylenglykol-400-Dimethacrylat vermischt und 100 Mikroliter 30%iges Wasserstoffperoxid wurden injiziert. Die Mischung wurde 30 min lang in einem Ultraschallbad evakuiert. Dann wurde die Mischung in ein offenes, flaches Gefäß geleert und dort bei 10°C 5 min lang mit UV- Licht (290 bis 400 nm, 40 mW/cm²) in einem Abstand von 5 cm bestrahlt.

Das erhaltene vernetzte Polymere wurde mit einem handelsüblichen flüssigen Biozid getränkt. Die erhaltene schwammartige Masse wurde in Streifen geschnitten, die auf Metallnetze aufgelegt wurden. Diese wurden in den Lüftungsschacht eines Krankenhausraumes eingelegt, wo sie die Bildung von Bakterien verhinderten.

## Patentansprüche

1. Verfahren zum Entfernen von schädlichen Mikroorganismen in der Luft durch Behandeln der Luft mit bioziden aktiven Agentien, die mit den Mikroorganismen reagieren und diese vernichten, **dadurch gekennzeichnet, dass** die aktiven Agentien in einer Matrix aus einem vernetzten, hydrophile Gruppen enthaltenden Polymeren verteilt sind und aus diesem langsam freigesetzt werden.

2. Verfahren nach Anspruch 1, **dadurch gekennzeichnet, dass** das vernetzte Polymere ein Kondensationsprodukt aus einem maleinisierten oder epoxidierten Polymeren und einem Vernetzer, vorzugsweise einem Polyamin, ist.

3. Verfahren nach Anspruch 1, **dadurch gekennzeichnet, dass** das vernetzte Polymere ein Copolymerisat aus einem monofunktionellen (Meth-)Acrylat-Monomeren und einem polyfunktionellen (Meth-)Acrylat- Monomeren ist.

4. Verfahren nach Anspruch 1, **dadurch gekennzeichnet, dass** die aktiven Agentien flüchtige, vorzugsweise bakterizid wirkende Substanzen sind, zum Beispiel Phenolverbindungen, Halogenverbindungen, quarternäre Ammoniumverbindungen, Aldehyde und stabilisiertes Wasserstoffperoxid, die mit dem Polymeren zusammen eine schwammartige Masse bilden, aus der sie langsam freigesetzt werden und mit den Mikroorganismen reagieren und diese vernichten.

5. Verfahren nach Anspruch 4, **dadurch gekennzeichnet, dass** die aktiven Agentien über einen Zeitraum von mindestens drei Wochen hinweg aus der schwammartigen Masse freigesetzt werden.

6. Verfahren nach Anspruch 4, **dadurch gekennzeichnet, dass** die aktiven Agentien in Mengen von 10 bis 90 Gew.% in der schwammartigen Masse enthalten sind.

7. Verfahren nach Anspruch 1, **dadurch gekennzeichnet, dass** die schwammartige Masse in Form von Kugeln, Spänen oder als Granulat zur Anwendung kommt.

8. Verfahren nach Anspruch 1, **dadurch gekennzeichnet, dass** die schwammartige Masse in Form von Krümeln, Platten oder Streifen mit einer Dicke von 0,2 bis 5 cm zur Anwendung kommt.

9. Verfahren nach Anspruch 8, **dadurch gekennzeichnet, dass** die Krümel, Platten oder Streifen auf Netze oder Gitter aufgelegt sind.

10. Verfahren nach Anspruch 9, **dadurch gekennzeichnet, dass** mehrere Gitter übereinander oder nebeneinander auf einem Gestell angeordnet sind, die in Ventilationskästen oder Klimakästen eingebaut sind.

11. Verfahren nach Anspruch 9, **dadurch gekennzeichnet, dass** die Netze oder Gitter in Lüftungsschächte eingelegt sind.
